# EUROPEAN PATENT APPLICATION

(11) **EP 3 058 952 A1**
(43) Date of publication of application: **24.08.2016**
(21) Application number: 16154327.7
(22) Date of filing: 06.04.2012
(51) Int. Cl.: A61K 39/00, C07K 16/24, A61K 47/02

(54) **FORMULATIONS WITH REDUCED VISCOSITY**

(30) Priority: 07.04.2011 US 201161473121 P
(62) Divisional of application: 12768307.6
(71) Applicant: GlaxoSmithKline LLC, Wilmington DE 19808 (US)
(72) Inventor: MONCK, Myrna A., King of Prussia, PA 19406 (US); WONG, Man Yi, King of Prussia, PA 19406 (US); ZHANG, Kai, King of Prussia, PA 19406 (US)
(74) Representative: Bhogal, Jasber Kaur

(57) **Abstract**

The present invention is directed to a method for reducing the viscosity of a formulation containing citrate and a therapeutic protein and formulations made using the claimed method.

SEQUENCE LISTING

<110> MONCK, Myrna A.
      WONG, Man Yi
      ZHANG, Kai

<120> FORMULATIONS WITH REDUCED VISCOSITY

<130> PU64606D1

<150> EP12768307.6
<151> 2012-04-06

<150> PCT/US2012/032462
<151> 2012-04-06

<150> 61/473,131
<151> 2011-04-07

<160> 2

<170> FastSEQ for Windows Version 4.0

<210> 1
<211> 449
<212> PRT
<213> Artificial Sequence

<220>
<223> Heavy Chain of anti-IL5 mAb

<400> 1
Gln Val Thr Leu Arg Glu Ser Gly Pro Ala Leu Val Lys Pro Thr Gln
 1               5                  10                  15
Thr Leu Thr Leu Thr Cys Thr Val Ser Gly Phe Ser Leu Thr Ser Tyr
            20                  25                  30
Ser Val His Trp Val Arg Gln Pro Pro Gly Lys Gly Leu Glu Trp Leu
        35                  40                  45
Gly Val Ile Trp Ala Ser Gly Gly Thr Asp Tyr Asn Ser Ala Leu Met
    50                  55                  60
Ser Arg Leu Ser Ile Ser Lys Asp Thr Ser Arg Asn Gln Val Val Leu
65                  70                  75                  80
Thr Met Thr Asn Met Asp Pro Val Asp Thr Ala Thr Tyr Tyr Cys Ala
                85                  90                  95
Arg Asp Pro Pro Ser Ser Leu Leu Arg Leu Asp Tyr Trp Gly Arg Gly
            100                 105                 110
Thr Pro Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Arg Val Glu Pro Lys Ser Cys Asp Lys
    210                 215                 220
Thr His Thr Cys Pro Pro Cys Pro Ala Pro Glu Leu Leu Gly Gly Pro

225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys

<210> 2
<211> 220
<212> PRT
<213> Artificial Sequence

<220>
<223> Light Chain of anti-IL5 mAb.

<400> 2
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Leu Leu Asn Ser
            20                  25                  30
Gly Asn Gln Lys Asn Tyr Leu Ala Trp Tyr Gln Gln Lys Pro Gly Gln
        35                  40                  45
Pro Pro Lys Leu Leu Ile Tyr Gly Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Asn
                85                  90                  95
Val His Ser Phe Pro Phe Thr Phe Gly Gly Gly Thr Lys Leu Glu Ile
            100                 105                 110
Lys Arg Thr Val Ala Ala Pro Ser Val Phe Ile Phe Pro Pro Ser Asp
        115                 120                 125
Glu Gln Leu Lys Ser Gly Thr Ala Ser Val Val Cys Leu Leu Asn Asn
    130                 135                 140
Phe Tyr Pro Arg Glu Ala Lys Val Gln Trp Lys Val Asp Asn Ala Leu
145                 150                 155                 160
Gln Ser Gly Asn Ser Gln Glu Ser Val Thr Glu Gln Asp Ser Lys Asp
                165                 170                 175
Ser Thr Tyr Ser Leu Ser Ser Thr Leu Thr Leu Ser Lys Ala Asp Tyr

                180                     185                     190
Glu Lys His Lys Val Tyr Ala Cys Glu Val Thr His Gln Gly Leu Ser
        195                     200                     205
Ser Pro Val Thr Lys Ser Phe Asn Arg Gly Glu Cys
    210                     215                     220

## Description

### Field of the Invention

The present invention relates to the field of formulations for therapeutic proteins. More specifically, the invention relates to formulations with reduced viscosity and methods of making the same.

### Background of the Invention

Many drug products that comprise proteins require high therapeutic doses to achieve an efficacious patient response. In order to attain therapeutic levels in the bloodstream, therapeutic proteins, including monoclonal antibodies, are required to be administered either via intravenous or subcutaneous injection due to their size and susceptibility to proteolytic degradation. Of these two routes of administration, subcutaneous injection is more convenient for patients since drug products targeting subcutaneous routes of administration can be given at home. There are a number of monoclonal antibody drug products that have been developed either de novo or as a product line extension in pre-filled syringes for a subcutaneous route of administration. Typically, not more than 1 mL of drug product solution can be administered as a single bolus dose via a prefilled syringe due to volume restrictions for dose administration in the subcutaneous space. However, the total volume and duration of administration is dictated by the concentration of the monoclonal antibody in the dosing solution. In order to achieve higher dose administration in smaller volumes, either for infusion or bolus administration, high concentrations of monoclonal antibodies in solution are required.

Many monoclonal antibodies in the concentration range exceeding 100mg/mL and most monoclonal antibodies at higher concentrations of 200mg/mL have relatively high viscosities leading to problems with the handling of the monoclonal antibody drug product solutions. Manufacturing processes such as tangential flow filtration for concentrating antibodies to high levels and sterile filtration are difficult and lead to yield losses for high viscosity solutions. Issues can also arise with handling and injectability of a drug product by patients or health care professionals when forces above approximately 20 Newtons must be achieved to deliver a subcutaneous dose of drug product using a prefilled syringe. It is clear that formulation approaches that give reductions in viscosity are required and the use of viscosity lowering excipients during formulation development is a viable approach.

### Summary of the Invention

The present invention is directed to a method for reducing the viscosity of a formulation containing citrate and a therapeutic protein.

In one embodiment the method comprises (a) providing a formulation comprising citrate; and (b) adding sodium chloride to the formulation to a concentration of about 20 mM to about 150 mM, wherein the viscosity of the formulation with the sodium chloride is reduced compared to the viscosity of the same formulation without sodium chloride.

In another embodiment the method comprises (a) providing a formulation comprising citrate; and (b) adding glycine and/or arginine to the formulation to a concentration of about 0.4% w/v to about 1.1% w/v, wherein the viscosity of the formulation with the glycine and/or arginine is reduced compared to the viscosity of the same formulation without glycine and/or arginine.

In another embodiment the method comprises (a) providing a formulation comprising citrate; and (b) adding phenylalanine to the formulation to a concentration of about 0.4% w/v to about 1.1% w/v, wherein the viscosity of the formulation with the phenylalanine is reduced compared to the viscosity of the same formulation without phenylalanine.

In another embodiment the method comprises (a) providing a formulation comprising citrate; and (b) adding tyrosine to the formulation to a concentration of about 0.001% w/v to about 0.005% w/v, wherein the viscosity of the formulation with the tyrosine is reduced compared to the viscosity of the same formulation without tyrosine.

In another embodiment the method comprises (a) providing a formulation comprising citrate; and (b) adding proline to the formulation to a concentration of about 4.0% w/v, wherein the viscosity of the formulation with the proline is reduced compared to the viscosity of the same formulation without proline.

The present invention is also directed to a stable formulation produced by any of the methods of the present invention.

The present invention is also directed to an article of manufacture comprising a container containing a formulation of the present invention.

### Brief Description of the Drawings

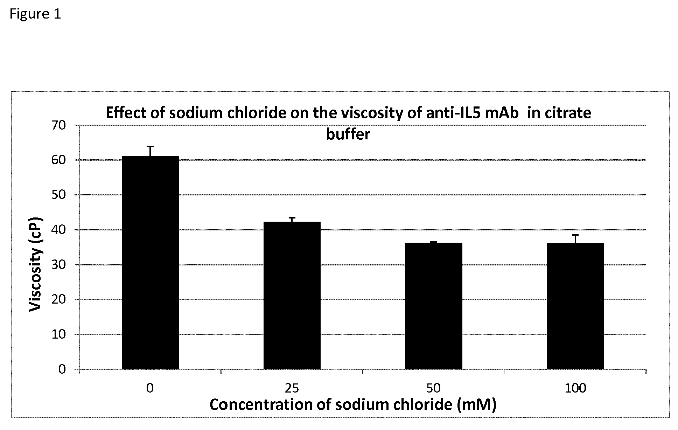

Figure 1. Sodium chloride: At all the concentrations tested, sodium chloride reduced the viscosity of anti-IL5 mAb in citrate buffer.

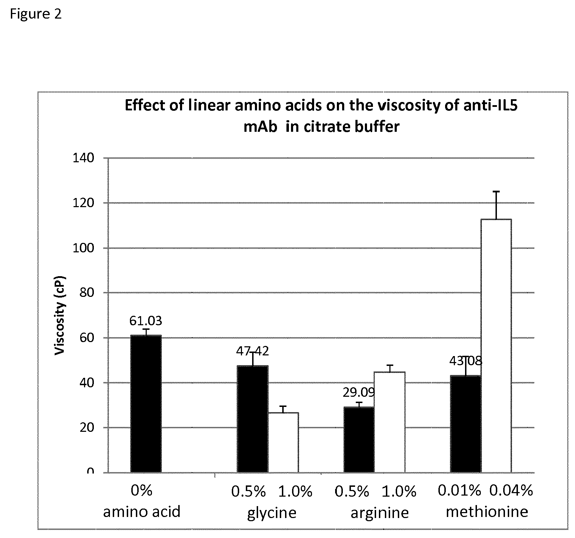

Figure 2. Citrate buffer: All concentrations of linear chain amino acids reduced the viscosity of the samples except 0.04% methionine.

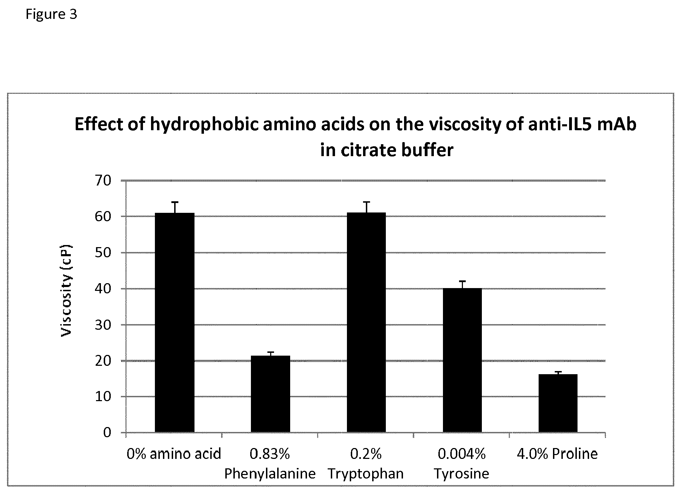

Figure 3. Citrate: Phenylalanine, tyrosine and proline reduced the viscosity of anti-IL5 mAb formulations but tryptophan did not change the viscosity of anti-IL5 mAb formulations.

### Detailed Description of the Invention

It is to be understood that this invention is not limited to particular methods, reagents, compounds, compositions, or biological systems, which can, of course, vary. It is also to be understood that the terminology used herein is for the purpose of describing particular embodiments only, and is not intended to be limiting. As used in this specification and the appended claims, the singular forms "a", "an", and "the" include plural referents unless the content clearly dictates otherwise. Thus, for example, reference to "a polypeptide" includes a combination of two or more polypeptides, and the like.

"About" as used herein when referring to a measurable value such as an amount, a temporal duration, and the like, is meant to encompass variations of ±20% or ±10%, including ±5%, ±1%, and ±0.1% from the specified value, as such variations are appropriate to perform the disclosed methods.

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

The present invention is directed to a method for reducing the viscosity of a formulation containing citrate and a therapeutic protein.

In exemplary embodiments of the present invention, the liquid polypeptide compositions that are produced exhibit desirable characteristics, such as desirable viscosity and surface tension characteristics.

The term "surface tension" refers to the attractive force exerted by the molecules below the surface upon those at the surface/air interface, resulting from the high molecular concentration of a liquid compared to the low molecular concentration of the gas. Liquids with low values of surface tension, such as nonpolar liquids, flow more readily than water. Typically, values of surface tensions are expressed in newtons/meters or dynes/centimeters.

"Dynamic surface tension" as referred to herein is the surface/air interface and the dynamic interfacial tension to the surface/surface interface. There are a number of alternative methods for measuring dynamic surface tension, for example, captive bubble surface tensionometry or pulsating bubble surface tensionometry.

The term "viscosity" refers to the internal resistance to flow exhibited by a fluid at a specified temperature; the ratio of shearing stress to rate of shear. A liquid has a viscosity of one poise if a force of 1 dyne/square centimeter causes two parallel liquid surfaces one square centimeter in area and one square centimeter apart to move past one another at a velocity of 1 cm/second. One poise equals one hundred centipoise.

When referring to apparent viscosity, it is understood that the value of viscosity is dependent on the conditions under which the measurement was taken, such as temperature, the rate of shear and the shear stress employed. The apparent viscosity is defined as the ratio of the shear stress to the rate of shear applied. There are a number of alternative methods for measuring apparent viscosity. For example, viscosity can be tested by a suitable cone and plate, parallel plate or other type of viscometer or rheometer.

In certain embodiments, the formulation with reduced viscosity has a viscosity less than about 50 cP, less than about 45 cP, less than about 40 cP, less than about 35 cP, less than about 30 cP, less than about 25 cP, less than about 20 cP, or less than about 15 cP.

"Polypeptide," "peptide" and "protein" are used interchangeably herein to refer to a polymer of amino acid residues. A polypeptide can be of natural (tissue-derived) origins, recombinant or natural expression from prokaryotic or eukaryotic cellular preparations, or produced chemically via synthetic methods. The terms apply to amino acid polymers in which one or more amino acid residue is an artificial chemical mimetic of a corresponding naturally occurring amino acid, as well as to naturally occurring amino acid polymers and non-naturally occurring amino acid polymer. Amino acid mimetics refers to chemical compounds that have a structure that is different from the general chemical structure of an amino acid, but that functions in a manner similar to a naturally occurring amino acid. Non-natural residues are well described in the scientific and patent literature; a few exemplary non-natural compositions useful as mimetics of natural amino acid residues and guidelines are described below. Mimetics of aromatic amino acids can be generated by replacing by, e.g., D- or L-naphylalanine; D- or L-phenylglycine; D- or L-2 thieneylalanine; D- or L-1, -2,3-, or 4-pyreneylalanine; D- or L-3 thieneylalanine; D- or L-(2-pyridinyl)-alanine; D- or L-(3-pyridinyl)-alanine; D- or L-(2-pyrazinyl)-alanine; D- or L-(4-isopropyl)-phenylglycine: D-(trifluoromethyl)-phenylglycine; D-(trifluoromethyl)-phenylalanine: D-p-fluoro-phenylalanine; D- or L-p-biphenylphenylalanine; K- or L-p-methoxy-biphenylphenylalanine: D- or L-2-indole(alkyl)alanines; and, D- or L-alkylainines, where alkyl can be substituted or unsubstituted methyl, ethyl, propyl, hexyl, butyl, pentyl, isopropyl, iso-butyl, sec-isotyl, iso-pentyl, or non-acidic amino acids. Aromatic rings of a non-natural amino acid include, e.g., thiazolyl, thiophenyl, pyrazolyl, benzimidazolyl, naphthyl, furanyl, pyrrolyl, and pyridyl aromatic rings.

"Peptide" as used herein includes peptides which are conservative variations of those peptides specifically exemplified herein. "Conservative variation" as used herein denotes the replacement of an amino acid residue by another, biologically similar residue. Examples of conservative variations include, but are not limited to, the substitution of one hydrophobic residue such as isoleucine, valine, leucine, alanine, cysteine, glycine, phenylalanine, proline, tryptophan, tyrosine, norleucine or methionine for another, or the substitution of one polar residue for another, such as the substitution of arginine for lysine, glutamic for aspartic acids, or glutamine for asparagine, and the like. Neutral hydrophilic amino acids which can be substituted for one another include asparagine, glutamine, serine and threonine. "Conservative variation" also includes the use of a substituted amino acid in place of an unsubstituted parent amino acid provided that antibodies raised to the substituted polypeptide also immunoreact with the unsubstituted polypeptide. Such conservative substitutions are within the definition of the classes of the peptides of the invention. "Cationic" as used herein refers to any peptide that possesses a net positive charge at pH 7.4. The biological activity of the peptides can be determined by standard methods known to those of skill in the art and described herein.

"Recombinant" when used with reference to a protein indicates that the protein has been modified by the introduction of a heterologous nucleic acid or protein or the alteration of a native nucleic acid or protein.

As used herein a "therapeutic protein" refers to any protein and/or polypeptide that can be administered to a mammal to elicit a biological or medical response of a tissue, system, animal or human that is being sought, for instance, by a researcher or clinician. A therapeutic protein may elicit more than one biological or medical response. Furthermore, the term "therapeutically effective amount" means any amount which, as compared to a corresponding subject who has not received such amount, results in, but is not limited to, healing, prevention, or amelioration of a disease, disorder, or side effect, or a decrease in the rate of advancement of a disease or disorder. The term also includes within its scope amounts effective to enhance normal physiological function as well as amounts effective to cause a physiological function in a patient which enhances or aids in the therapeutic effect of a second pharmaceutical agent.

All "amino acid" residues identified herein are in the natural L-configuration. In keeping with standard polypeptide nomenclature, abbreviations for amino acid residues are as shown in the following table.

**Table 1. Amino acid abbreviations.**

| 1 Letter | 3 Letter | Amino Acid |
|---|---|---|
| Y | Tyr | L-tyrosine |
| G | Gly | L-glycine |
| F | Phe | L-phenylalanine |
| M | Met | L-methionine |
| A | Ala | L-alanine |
| S | Ser | L-serine |
| I | Ile | L-isoleucine |
| L | Leu | leucine |
| T | Thr | L-threonine |
| V | Val | L-valine |
| P | Pro | L-proline |
| K | Lys | L-lysine |
| H | His | L-histidine |
| Q | Gln | L-glutamine |
| E | Glu | L-glutamic acid |
| W | Trp | L-tryptophan |
| R | Arg | L-arginine |
| D | Asp | L-aspartic acid |
| N | Asn | L-asparagine |
| C | Cys | L-cysteine. |

It should be noted that all amino acid residue sequences are represented herein by formulae whose left to right orientation is in the conventional direction of amino-terminus to carboxy-terminus.

In another embodiment the polypeptide is an antigen binding polypeptide. In one embodiment the antigen binding polypeptide is selected from the group consisting of a soluble receptor, antibody, antibody fragment, immunoglobulin single variable domain, Fab, F(ab')2, Fv, disulphide linked Fv, scFv, closed conformation multispecific antibody, disulphide-linked scFv, or diabody.

The term "antigen binding polypeptide" as used herein refers to antibodies, antibody fragments and other protein constructs which are capable of binding to an antigen.

The terms Fv, Fc, Fd, Fab, or F(ab)2 are used with their standard meanings (see, e.g., Harlow et al., Antibodies A Laboratory Manual, Cold Spring Harbor Laboratory, (1988)).

A "chimeric antibody" refers to a type of engineered antibody which contains a naturally-occurring variable region (light chain and heavy chains) derived from a donor antibody in association with light and heavy chain constant regions derived from an acceptor antibody.

A "humanized antibody" refers to a type of engineered antibody having its CDRs derived from a non-human donor immunoglobulin, the remaining immunoglobulin-derived parts of the molecule being derived from one (or more) human immunoglobulin(s). In addition, framework support residues may be altered to preserve binding affinity (see, e.g., Queen et al., Proc. Natl. Acad Sci USA, 86:10029-10032 (1989), Hodgson et al., Bio/Technology, 9:421 (1991)). A suitable human acceptor antibody may be one selected from a conventional database, e.g., the KABAT.RTM. database, Los Alamos database, and Swiss Protein database, by homology to the nucleotide and amino acid sequences of the donor antibody. A human antibody characterized by a homology to the framework regions of the donor antibody (on an amino acid basis) may be suitable to provide a heavy chain constant region and/or a heavy chain variable framework region for insertion of the donor CDRs. A suitable acceptor antibody capable of donating light chain constant or variable framework regions may be selected in a similar manner. It should be noted that the acceptor antibody heavy and light chains are not required to originate from the same acceptor antibody. The prior art describes several ways of producing such humanized antibodies-see for example EP-A-0239400 and EP-A-054951.

The term "donor antibody" refers to an antibody (monoclonal, and/or recombinant) which contributes the amino acid sequences of its variable regions, CDRs, or other functional fragments or analogs thereof to a first immunoglobulin partner, so as to provide the altered immunoglobulin coding region and resulting expressed altered antibody with the antigenic specificity and neutralizing activity characteristic of the donor antibody.

The term "acceptor antibody" refers to an antibody (monoclonal and/or recombinant) heterologous to the donor antibody, which contributes all (or any portion, but in some embodiments all) of the amino acid sequences encoding its heavy and/or light chain framework regions and/or its heavy and/or light chain constant regions to the first immunoglobulin partner. In certain embodiments a human antibody is the acceptor antibody.

"CDRs" are defined as the complementarity determining region amino acid sequences of an antibody which are the hypervariable regions of immunoglobulin heavy and light chains. See, e.g., Kabat et al., Sequences of Proteins of Immunological Interest, 4th Ed., U.S. Department of Health and Human Services, National Institutes of Health (1987). There are three heavy chain and three light chain CDRs (or CDR regions) in the variable portion of an immunoglobulin. Thus, "CDRs" as used herein refers to all three heavy chain CDRs, or all three light chain CDRs (or both all heavy and all light chain CDRs, if appropriate). The structure and protein folding of the antibody may mean that other residues are considered part of the antigen binding region and would be understood to be so by a skilled person. See for example Chothia et al., (1989) Conformations of immunoglobulin hypervariable regions; Nature 342, p 877-883.

As used herein the term "domain" refers to a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. An "antibody single variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

The phrase "immunoglobulin single variable domain" refers to an antibody variable domain (V_{H}, V_{HH}, V_{L}) that specifically binds an antigen or epitope independently of a different V region or domain. An immunoglobulin single variable domain can be present in a format (e.g., homo- or hetero-multimer) with other, different variable regions or variable domains where the other regions or domains are not required for antigen binding by the single immunoglobulin variable domain (i.e., where the immunoglobulin single variable domain binds antigen independently of the additional variable domains). A "domain antibody" or "dAb" is the same as an "immunoglobulin single variable domain" which is capable of binding to an antigen as the term is used herein. An immunoglobulin single variable domain may be a human antibody variable domain, but also includes single antibody variable domains from other species such as rodent (for example, as disclosed in WO 00/29004), nurse shark and Camelid V_{HH} dAbs (nanobodies). Camelid V_{HH} are immunoglobulin single variable domain polypeptides that are derived from species including camel, Ilama, alpaca, dromedary, and guanaco, which produce heavy chain antibodies naturally devoid of light chains. Such V_{HH} domains may be humanized according to standard techniques available in the art, and such domains are still considered to be "domain antibodies" according to the invention. As used herein "V_{H} includes camelid V_{HH} domains. NARV are another type of immunoglobulin single variable domain which were identified in cartilaginous fish including the nurse shark. These domains are also known as Novel Antigen Receptor variable region (commonly abbreviated to V(NAR) or NARV). For further details see Mol. Immunol. 44, 656-665 (2006) and US20050043519A.

The term "Epitope-binding domain" refers to a domain that specifically binds an antigen or epitope independently of a different V region or domain, this may be a domain antibody (dAb), for example a human, camelid or shark immunoglobulin single variable domain.

As used herein, the term "antigen-binding site" refers to a site on a protein which is capable of specifically binding to antigen, this may be a single domain, for example an epitope-binding domain, or it may be paired V_{H}/V_{L} domains as can be found on a standard antibody. In some aspects of the invention single-chain Fv (ScFv) domains can provide antigen-binding sites.

The terms "mAbdAb" and dAbmAb" are used herein to refer to antigen-binding proteins of the present invention. The two terms can be used interchangeably, and are intended to have the same meaning as used herein.

In one embodiment the method comprises (a) providing a formulation comprising citrate; and (b) adding sodium chloride to the formulation to a concentration of about 20 mM to about 150 mM, wherein the viscosity of the formulation with the sodium chloride is reduced compared to the viscosity of the same formulation without sodium chloride. In certain embodiments, the sodium chloride is added to a concentration selected from the group consisting of about 25 mM, about 50 mM, and about 100mM. In certain embodiments, the viscosity of the formulation with sodium chloride is reduced by at least about 5%, at least about 10%, at least about 15%, at least about 20%, at least about 25%, at least about 30%, or at least about 40% compared to the viscosity of the formulation in the absence of sodium chloride. In certain embodiments, the viscosity of the formulation with sodium chloride is less than about 50 cP, less than about 45 cP, less than about 40 cP, or less than about 35 cP.

In one embodiment the method comprises (a) providing a formulation comprising citrate; and (b) adding an amino acid or multiple amino acids to the formulation, wherein the viscosity of the formulation with the amino acid(s) is reduced compared to the viscosity of the same formulation without the same amino acids(s). In certain embodiments the amino acid(s) is a linear amino acid. In other embodiments the amino acid comprises a cyclic portion. In one embodiment the amino acid(s) is tyrosine, glycine, phenylalanine, methionine, alanine, serine, isoleucine, leucine, threonine, valine, proline, lysine, histidine, glutamine, glutamic acid, arginine, aspartic acid, asparagine, cysteine.

In one embodiment the method comprises (a) providing a formulation comprising citrate; and (b) adding glycine and/or arginine to the formulation to a concentration of about 0.4% w/v to about 1.1% w/v, wherein the viscosity of the formulation with the glycine and/or arginine is reduced compared to the viscosity of the same formulation without glycine and/or arginine. In certain embodiments, the glycine and/or arginine is added to a concentration selected from the group consisting of about 0.5% w/v and about 1% w/v. In certain embodiments, the viscosity of the formulation with glycine and/or arginine is reduced by at least about 5%, at least about 10%, at least about 15%, at least about 20%, at least about 25%, at least about 30%, at least about 40%, or at least about 50% compared to the viscosity of the formulation in the absence of glycine and/or arginine. In certain embodiments, the viscosity of the formulation with glycine and/or arginine is less than about 50 cP, less than about 45 cP, less than about 40 cP, less than about 35 cP, less than about 30 cP, or less than about 25 cP.

In another embodiment the method comprises (a) providing a formulation comprising citrate; and (b) adding phenylalanine to the formulation to a concentration of about 0.4% w/v to about 1.1% w/v, wherein the viscosity of the formulation with the phenylalanine is reduced compared to the viscosity of the same formulation without phenylalanine. In certain embodiments, the phenylalanine is added to a concentration of about 0.8% w/v. In certain embodiments, the viscosity of the formulation with phenylalanine is reduced by at least about 5%, at least about 10%, at least about 15%, at least about 20%, at least about 25%, at least about 30%, at least about 40%, or at least about 50% compared to the viscosity of the formulation in the absence of phenylalanine. In certain embodiments, the viscosity of the formulation with phenylalanine is less than about 50 cP, less than about 45 cP, less than about 40 cP, less than about 35 cP, less than about 30 cP, or less than about 25 cP.

In another embodiment the method comprises (a) providing a formulation comprising citrate; and (b) adding tyrosine to the formulation to a concentration of about 0.001% w/v to about 0.005% w/v, wherein the viscosity of the formulation with the tyrosine is reduced compared to the viscosity of the same formulation without tyrosine. In certain embodiments, the tyrosine is added to a concentration of about 0.004% w/v. In certain embodiments, the viscosity of the formulation with tyrosine is reduced by at least about 5%, at least about 10%, at least about 15%, at least about 20%, at least about 25%, at least about 30%, at least about 40%, or at least about 50% compared to the viscosity of the formulation in the absence of tyrosine. In certain embodiments, the viscosity of the formulation with tyrosine is less than about 50 cP, less than about 45 cP, less than about 40 cP, less than about 35 cP, less than about 30 cP, or less than about 25 cP.

In another embodiment the method comprises (a) providing a formulation comprising citrate; and (b) adding methionine to the formulation to a concentration of about 0.01% w/v. In certain embodiments, the viscosity of the formulation with methionine is reduced by at least about 5%, at least about 10%, at least about 15%, at least about 20%, at least about 25%, at least about 30%, compared to the viscosity of the formulation in the absence of methionine. In certain embodiments, the viscosity of the formulation with methionine is less than about 50 cP, less than about 45 cP, less than about 40 cP, or less than about 35 cP.

In another embodiment the method comprises (a) providing a formulation comprising citrate; and (b) adding proline to the formulation to a concentration of about 4.0%w/v, wherein the viscosity of the formulation with the proline is reduced compared to the viscosity of the same formulation without proline. In certain embodiments, the viscosity of the formulation with proline is reduced by at least about 5%, at least about 10%, at least about 15%, at least about 20%, at least about 25%, at least about 30%, at least about 40%, at least about 50%, at least about 60%, at least about 70%, or at least about 75% compared to the viscosity of the formulation in the absence of proline. In certain embodiments, the viscosity of the formulation with proline is less than about 50 cP, less than about 45 cP, less than about 40 cP, less than about 35 cP, less than about 30 cP, less than about 25 cP, less than about 20 cP, or less than about 15 cP.

In another embodiment the method further comprises determining the stability of the protein formulation.

In another embodiment the formulation further comprises additional excipients. "Excipients" includes, but is not limited to, stabilizers, for example, human serum albumin (hsa), bovine serum albumin (bsa), α-casein, globulins, α-lactalbumin, LDH, lysozyme, myoglobin, ovalbumin, RNase A; buffering agents, for example, citric acid, HEPES, histidine, potassium acetate, postassium citrate, potassium phosphate (KH₂PO₄), sodium acetate, sodium bicarbonate, sodium citrate, sodium phosphate (NAH₂PO₄), Tris base, and Tris-HCl; amino acids/metabolites, for example, glycine, alanine (α-alanine, β-alanine), arginine, betaine, leucine, lysine, glutamic acid, aspartic acid, histidine, proline, 4-hydroxyproline, sarcosine, γ-aminobutyric acid (GABA), opines (alanopine, octopine, strombine), and trimethylamine N-oxide (TMAO); surfactants, for example, polysorbate 20 and 80, and poloxamer 407: lipid molecules, for example, phosphatidyl choline, ethanolamine, and acethyltryptophanate: polymers, for example, polyethylene glycol (PEG), and polyvinylpyrrolidone (PVP) 10, 24, 40; low molecular weight excipients, for example, arabinose, cellobiose, ethylene glycol, fructose, fucose, galactose, glycerin/glycerol, glucose, inositol, lactose, mannitol, maltose, maltotriose, mannose, melibiose, 2-methyl-2,4-pentanediol, octulose, propylene glycol, raffinose, ribose, sorbitol, sucrose, trehalose, xylitol, and xylose; and high molecular weight excipients, for example, cellulose, β-cyclodextrin, dextran (10 kd), dextran (40 kd), dextran (70 kd), ficoll, gelatin, hydroxypropylmethyl-cellulose, hydroxyethyl starch, maltodextrin, methocel, peg (6 kd), polydextrose, polyvinylpyrrolidone (PVP) k15 (10 kd), PVP (40 kd), PVP k30 (40 kd), PVP k90 (1000 kd), sephadex G 200, and starch; antioxidants, for example, ascorbic acid, cysteine HCl, thioglycerol, thioglycolic acid, thiosorbitol, and glutathione; reducing agents, for example, cysteine HCl, dithiothreotol, and other thiol or thiophenes; chelating agents, for example, EDTA, EGTA, glutamic acid, and aspartic acid; inorganic salts/metals, for example, Ca²⁺, Ni²⁺, Mg²⁺, Mn²⁺, Na₂SO₄, (NH₄)₂SO₄, Na₂HPO₄/NaH₂PO₄, K₂HPO₄/KH₂PO₄, MgSO₄, and NaF; organic salts, for example, Na acetate, Na polyethylene, Na caprylate (Na octanoate), proprionate, lactate, succinate, and citrate; organic solvents, for example, acetonitrile, dimethylsulfoxide (dmso), and ethanol.

In one embodiment the formulation further comprises sucrose. In one embodiment the formulation comprises sucrose at a concentration of about 150 to about 300 mM. In one embodiment the formulation comprises sucrose at a concentration of about 200 to about 250 mM. In one embodiment the formulation comprises sucrose at a concentration of about 234 mM.

In one embodiment the formulation is formulated to a pH of about 5.0 to about 8.0. In one embodiment the formulation is formulated to a pH of about 6.0. In one embodiment the formulation comprises about 10 mM to about 50 mM citrate. In one embodiment the formulation comprises about 20 mM citrate.

In another embodiment the formulation further comprises polysorbate-80. In one embodiment the formulation further comprises polysorbate-80 at a concentration of up to 0.05% w/v.

In another embodiment the therapeutic protein is an antigen binding polypeptide. In one embodiment the antigen binding polypeptide is an antibody. In one embodiment the antigen binding polypeptide is an immunoglobulin single variable domain. In one embodiment the antigen binding polypeptide binds to interleukin 5 (IL5). In one embodiment the antigen binding polypeptide is an anti-IL5 antibody. In one embodiment the anti-IL5 antibody comprises a heavy chain comprising SEQ ID NO:1 and a light chain comprising SEQ ID NO:2.

In another embodiment the therapeutic protein is present at a concentration of at least about 150 mg/ml, at least about 175 mg/ml, at least about 200 mg/ml, at least about 225 mg/ml, at least about 250 mg/ml, at least about 275 mg/ml, or at least about 300 mg/ml. In another embodiment the therapeutic protein is present at a concentration of at least about 150 mg/ml to about 300 mg/ml. In one embodiment the therapeutic protein is present at a concentration of about 200 mg/ml.

In one embodiment the formulation is lyophilized or spray dried, and then reconstituted before the viscosity is determined. In certain embodiments the formulation with reduced viscosity is lyophilized or spray dried and then later reconstituted with a dispersing agent. In one embodiment the dispersing agent is sterile water or "water for injection" (WFI). The liquid polypeptide can be further diluted with isotonic saline or other excipients to produce a desirable concentration prior to administration. In one embodiment the formulation is a reconstituted formulation. In another embodiment the formulation is a liquid pharmaceutical formulation.

The agents used to reduce viscosity can be added at any stage of the formulation process. For example, before, after, or concurrently with the citrate, the therapeutic protein, or with any excipients.

The formulations of the present invention may be administered by any suitable route of administration, including systemic administration. Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient's lungs whether inhaled through the mouth or through the nasal passages.

The present invention is also directed to a stable formulation produced by any of the methods of the present invention.

In one embodiment the formulation comprises citrate, the therapeutic protein, and sodium chloride. In one embodiment the concentration of sodium chloride is about 20 mM to about 150 mM, wherein the viscosity of the formulation with the sodium chloride is reduced compared to the viscosity of the same formulation without sodium chloride. In certain embodiments, the concentration of sodium chloride is about 25 mM, about 50 mM, or about 100mM. In certain embodiments, the viscosity of the formulation with sodium chloride is reduced by at least about 5%, at least about 10%, at least about 15%, at least about 20%, at least about 25%, at least about 30%, or at least about 40% compared to the viscosity of the formulation in the absence of sodium chloride. In certain embodiments, the viscosity of the formulation with sodium chloride is less than about 50 cP, less than about 45 cP, less than about 40 cP, or less than about 35 cP.

In one embodiment the formulation comprises citrate, the therapeutic protein, and an amino acid or multiple amino acids, wherein the viscosity of the formulation with the amino acid(s) is reduced compared to the viscosity of the same formulation without the same amino acids(s). In certain embodiments the amino acid(s) is a linear amino acid. In other embodiments the amino acid comprises a cyclic portion. In one embodiment the amino acid(s) is tyrosine, glycine, phenylalanine, methionine, alanine, serine, isoleucine, leucine, threonine, valine, proline, lysine, histidine, glutamine, glutamic acid, arginine, aspartic acid, asparagine, cysteine.

In one embodiment the formulation comprises citrate, the therapeutic protein, and glycine and/or arginine. In one embodiment the concentration of glycine and/or arginine is about 0.4% w/v to about 1.1% w/v, wherein the viscosity of the formulation with the glycine and/or arginine is reduced compared to the viscosity of the same formulation without glycine and/or arginine. In one embodiment the concentration of glycine and/or arginine is about 0.5% w/v or about 1% w/v. In certain embodiments, the viscosity of the formulation with glycine and/or arginine is reduced by at least about 5%, at least about 10%, at least about 15%, at least about 20%, at least about 25%, at least about 30%, at least about 40%, or at least about 50% compared to the viscosity of the formulation in the absence of glycine and/or arginine. In certain embodiments, the viscosity of the formulation with glycine and/or arginine is less than about 50 cP, less than about 45 cP, less than about 40 cP, less than about 35 cP, less than about 30 cP, or less than about 25 cP.

In one embodiment the formulation comprises citrate, the therapeutic protein, and phenylalanine. In one embodiment the concentration of phenylalanine is about 0.4% w/v to about 1.1% w/v, wherein the viscosity of the formulation with the phenylalanine is reduced compared to the viscosity of the same formulation without phenylalanine. In certain embodiments, the concentration of phenylalanine is about 0.8% w/v. In certain embodiments, the viscosity of the formulation with phenylalanine is reduced by at least about 5%, at least about 10%, at least about 15%, at least about 20%, at least about 25%, at least about 30%, at least about 40%, or at least about 50% compared to the viscosity of the formulation in the absence of phenylalanine. In certain embodiments, the viscosity of the formulation with phenylalanine is less than about 50 cP, less than about 45 cP, less than about 40 cP, less than about 35 cP, less than about 30 cP, or less than about 25 cP.

In one embodiment the formulation comprises citrate, the therapeutic protein, and tyrosine. In one embodiment the concentration of tyrosine is about 0.001% w/v to about 0.005% w/v, wherein the viscosity of the formulation with the tyrosine is reduced compared to the viscosity of the same formulation without tyrosine. In certain embodiments, the concentration of tyrosine is about 0.004% w/v. In certain embodiments, the viscosity of the formulation with tyrosine is reduced by at least about 5%, at least about 10%, at least about 15%, at least about 20%, at least about 25%, at least about 30%, at least about 40%, or at least about 50% compared to the viscosity of the formulation in the absence of tyrosine. In certain embodiments, the viscosity of the formulation with tyrosine is less than about 50 cP, less than about 45 cP, less than about 40 cP, less than about 35 cP, less than about 30 cP, or less than about 25 cP.

In one embodiment the formulation comprises citrate, the therapeutic protein, and methionine. In one embodiment the concentration of methionine is about 0.01% w/v, wherein the viscosity of the formulation with the methionine is reduced compared to the viscosity of the same formulation without tyrosine. In certain embodiments, the viscosity of the formulation with methionine is reduced by at least about 5%, at least about 10%, at least about 15%, at least about 20%, at least about 25%, at least about 30%, compared to the viscosity of the formulation in the absence of methionine. In certain embodiments, the viscosity of the formulation with methionine is less than about 50 cP, less than about 45 cP, less than about 40 cP, or less than about 35 cP.

In one embodiment the formulation comprises citrate, the therapeutic protein, and proline. In one embodiment the concentration of proline is about 4.0% w/v, wherein the viscosity of the formulation with the proline is reduced compared to the viscosity of the same formulation without proline. In certain embodiments, the viscosity of the formulation with proline is reduced by at least about 5%, at least about 10%, at least about 15%, at least about 20%, at least about 25%, at least about 30%, at least about 40%, at least about 50%, at least about 60%, at least about 70%, or at least about 75% compared to the viscosity of the formulation in the absence of proline. In certain embodiments, the viscosity of the formulation with proline is less than about 50 cP, less than about 45 cP, less than about 40 cP, less than about 35 cP, less than about 30 cP, less than about 25 cP, less than about 20 cP, or less than about 15 cP.

In one embodiment the formulation further comprises sucrose. In one embodiment the formulation comprises sucrose at a concentration of about 150 to about 300 mM. In one embodiment the formulation comprises sucrose at a concentration of about 200 to about 250 mM. In one embodiment the formulation comprises sucrose at a concentration of about 234 mM.

In one embodiment the formulation is formulated to a pH of about 5.0 to about 8.0. In one embodiment the formulation is formulated to a pH of about 6.0. In one embodiment the formulation comprises about 10 mM to about 50 mM citrate. In one embodiment the formulation comprises about 20 mM citrate.

In another embodiment the formulation further comprises polysorbate-80. In another embodiment the formulation further comprises polysorbate-80. In one embodiment the formulation further comprises polysorbate-80 at a concentration of up to 0.05% w/v.

The present invention is also directed to an article of manufacture comprising a container containing a formulation of the present invention. In one embodiment the article of manufacture further comprises directions for administration of the formulation.

### Examples

Glycine, tyrosine, tryptophan, phenylalanine, and proline were acquired from Sigma-Aldrich. Arginine was acquired from MP-Biomedicals and methionine was acquired from JT Baker. All the amino acids were laboratory grade. Anti-IL5 mAb stock (220 mg/mL) solutions were prepared in-house and were formulated with 234mM sucrose in citrate buffer (pH 6.0).

The concentration of the anti-IL5 mAb solution was adjusted to 200 mg/mL for viscosity measurements as described below. For sodium chloride, glycine, arginine, methionine and tyrosine, stock solutions were prepared in citrate buffers (Tables 2a and b) and spiked into the 220mg/mL anti-IL5 mAb stock solution of the respective buffer (Tables 3a and b).

For tryptophan, phenylalanine, and proline the amino acids were dissolved directly into the anti-IL5 mAb solution so as to attain the targeted amino acid concentration in Table 3b. The concentrations could not be attained by making a stock solution due to their low water solubility.

**Table 2a: Concentration of stock solution of salt.**

| Name of salt | Concentration of stock solution in citrate buffers (M) |
|---|---|
| Sodium chloride | 2.0 |

**Table 2b: Concentrations of stock solutions of amino acids.**

| Name of amino acid | Concentration of stock solution in citrate buffers (% w/v) |
|---|---|
| Glycine | 10.90 |
| Arginine | 10.90 |
| Methionine | 0.80 |
| Tyrosine | 0.04 |

**Table 3a: Dilution scheme of salt to attain 200 mg/mL anti-IL5 mAb with salt.**

| | Salt concentration in the final 200mg/mL anti-IL5 mAb solution (mM) | Volume of 220mg/mL anti-IL5 mAb stock (µL) | Volume of salt stock (µL) | Volume of citrate buffer (µL) |
|---|---|---|---|---|
| Sodium chloride | 100 | 4545 | 250 | 205 |
| Sodium chloride | 50 | 4545 | 125 | 330 |
| Sodium chloride | 25 | 4545 | 62.5 | 392.5 |

**Table 3b: Dilution scheme of amino acids to attain 200 mg/mL anti-IL5 mAb with the amino acid concentrations.**

| | Amino acid concentration in the final 200mg/mL anti-IL5 mAb solution (% w/v) | Volume of 220mg/mL anti-IL5 mAb stock (µL) | Volume of amino acid stock (µL) | Volume of citrate buffer (µL) | Weight of amino acid (g) |
|---|---|---|---|---|---|
| Glycine | 0.5 | 1818 | 91 | 91 | NA |
| Glycine | 1.0 | 1818 | 182 | 0 | NA |
| Arginine | 0.5 | 1818 | 91 | 91 | NA |
| Arginine | 1.0 | 1818 | 182 | 0 | NA |
| Methionine | 0.01 | 1818 | 25 | 157 | NA |
| Methionine | 0.04 | 1818 | 100 | 82 | NA |
| Tryptophan | 0.2 | 9090 | NA | 910 | 0.05 |
| Phenylalanine | 0.83 | 5454 | NA | 546 | 0.02 |
| Tyrosine | 0.004 | 1818 | 182 | 0 | NA |
| Proline | 4.0 | 9090 | NA | 900 | 0.4 |

Following sample dilution, the viscosity of the samples was measured with a Brookfield LVDVUUItra III C/P rheometer at 25 °C. The spindle used was CP-40 and 500 µL of sample was loaded for each measurement. Mean viscosity values were calculated from viscosity values obtained that were unchanged with increases in % torque.

## Claims

1. A method for reducing the viscosity of a formulation containing citrate and a therapeutic protein, the method comprising; (a) providing a formulation comprising citrate; and
(b) (i) adding sodium chloride to the formulation to a concentration of about 20 mM to about 150 mM, wherein the viscosity of the formulation with the sodium chloride is reduced compared to the viscosity of the same formulation without sodium chloride; or
(b)(ii) adding glycine and/or arginine to the formulation to a concentration of about 0.4% w/v to about 1.1% w/v, wherein the viscosity of the formulation with the glycine and/or arginine is reduced compared to the viscosity of the same formulation without glycine and/or arginine; or
(b)(iii) adding phenylalanine to the formulation to a concentration of about 0.4% w/v to about 1.1% w/v, wherein the viscosity of the formulation with the phenylalanine is reduced compared to the viscosity of the same formulation without phenylalanine; or
(b)(iv) adding tyrosine to the formulation to a concentration of about 0.001% w/v to about 0.005% w/v, wherein the viscosity of the formulation with the tyrosine is reduced compared to the viscosity of the same formulation without tyrosine; or
(b)(v) adding proline to the formulation to a concentration of about 4.0% w/v, wherein the viscosity of the formulation with the proline is reduced compared to the viscosity of the same formulation without proline.

2. The method of claim 1 wherein
(i) the sodium chloride is added to a concentration selected from the group consisting of about 25 mM, about 50 mM, and about 100mM; or
(ii) the glycine and/or arginine is added to a concentration selected from the group consisting of about 0.5% w/v and about 1% w/v; or
(iii) the phenylalanine is added to a concentration of about 0.8% w/v; or
(iv) the tyrosine is added to a concentration of about 0.004% w/v.

3. The method of any preceding claim, wherein the viscosity of the formulation with
(a) (i) sodium chloride is reduced by at least about 5%, at least about 10%, at least about 15%, at least about 20%, at least about 25%, at least about 30%, or at least about 40% compared to the viscosity of the formulation in the absence of sodium chloride; or
(b) (ii) glycine and/or arginine, or (iii) phenylalanine, or (iv) tyrosine, is reduced by at least about 5%, at least about 10%, at least about 15%, at least about 20%, at least about 25%, at least about 30%, at least about 40%, or at least about 50% compared to the viscosity of the formulation in the absence of (ii) glycine and/or arginine, or (iii) phenylalanine, or (iv) tyrosine; or
(c) (v) proline is reduced by at least about 5%, at least about 10%, at least about 15%, at least about 20%, at least about 25%, at least about 30%, at least about 40%, at least about 50%, at least about 60%, at least about 70%, or at least about 75% compared to the viscosity of the formulation in the absence of proline.

4. The method of any preceding claim, wherein the viscosity of the formulation with
(a) (i) sodium chloride is less than about 50 cP, less than about 45 cP, less than about 40 cP, or less than about 35 cP; or
(b) (ii) glycine and/or arginine, or (iii) phenylalanine, or (iv) tyrosine, is less than about 50 cP, less than about 45 cP, less than about 40 cP, less than about 35 cP, less than about 30 cP, or less than about 25 cP; or
(c) (v) proline is less than about 50 cP, less than about 45 cP, less than about 40 cP, less than about 35 cP, less than about 30 cP, less than about 25 cP, less than about 20 cP, or less than about 15 cP.

5. The method of any preceding claim, wherein the method further comprises determining the stability of the protein formulation.

6. The method of any preceding claim, wherein the formulation further comprises sucrose.

7. The method of claim 6, wherein the formulation further comprises sucrose at a concentration of about 234 mM.

8. The method of any preceding claim, wherein the formulation is formulated to a pH of about 6.0.

9. The method of any preceding claim, wherein the formulation further comprises polysorbate-80.

10. The method of any preceding claim wherein the therapeutic protein is an antigen binding polypeptide.

11. The method of any preceding claim wherein the antigen binding polypeptide is an antibody.

12. The method of any preceding claim wherein the antigen binding polypeptide is an immunoglobulin single variable domain.

13. The method of claim 10 where the antigen binding polypeptide binds to interleukin 5 (IL5).

14. The method of claim 13, wherein the antigen binding polypeptide is an anti-IL5 antibody.
